# EUROPEAN PATENT SPECIFICATION

(11) **EP 4 147 767 B1**
(45) Date of publication and mention of the grant of the patent: **18.06.2025**
(21) Application number: 21854277.7
(22) Date of filing: 17.05.2021
(51) Int. Cl.: B01D 53/26, A61L 9/014, F24F 3/14, B01D 53/06, B01D 53/04

(54) **AIR CONDITIONING ROTATING BODY AND AIR TREATMENT DEVICE**
KLIMAANLAGENDREHKÖRPER UND LUFTBEHANDLUNGSVORRICHTUNG
CORPS ROTATIF DE CLIMATISATION ET DISPOSITIF DE TRAITEMENT D'AIR

(30) Priority: 07.08.2020 JP 2020134346
(43) Date of publication of application: 15.03.2023
(73) Proprietor: Daikin Industries, Ltd., Osaka-shi, Osaka 530-8323 (JP)
(72) Inventor: OHDOU, Tsunahiro, Osaka-shi, Osaka 530-8323 (JP); TAKAHASHI, Takashi, Osaka-shi, Osaka 530-8323 (JP); OKUBO, Eisaku, Osaka-shi, Osaka 530-8323 (JP); TANAKA, Hidekazu, Osaka-shi, Osaka 530-8323 (JP)
(74) Representative: Goddar, Heinz J.
(86) International application number: PCT/JP2021/018600
(87) International publication number: WO 2022/030067

(56) References cited:
- JP-A- 2004 100 940
- JP-A- 2004 160 444
- JP-A- 2004 160 444
- JP-A- 2004 321 964
- JP-A- 2005 211 811
- JP-A- 2006 052 922
- JP-A- 2007 007 536
- JP-A- S5 325 958
- US-A1- 2004 231 179

## Description

### TECHNICAL FIELD

The present disclosure relates to an air conditioning rotating body and an air treatment device.

### BACKGROUND ART

There is an air treatment device in which a rotor made of a material with excellent heat storage properties, such as aluminum and stainless steel, is rotated about a shaft, with a portion of the rotor placed in an exhaust passage and the other portion of the rotor placed in an air supply passage. Such an air treatment device is used as a heat exchanger configured to exchange heat between exhaust air and supply air.

There is also an air treatment device in which a rotor having a honeycomb structure on which an adsorbent, such as zeolite and porous silica, is supported is rotated about a shaft, and which is configured such that treated air passes through a portion of the rotor and regenerated air passes through the other portion of the rotor. Such an air treatment device is used for humidity control, deodorization, and other purposes.

An air treatment device using a rotor is configured such that the rotor is housed in a casing so as to be freely rotatable, and that sealing structures are provided along the outer circumference and diameter of the rotor to define an air passage. Patent Document 1 discloses, as such sealing structures, an air leakage preventing member that protrudes in the axial direction from the outer circumferential surface of the rotor made of a heat exchange material, and a receiving member that protrudes from the inner surface of the casing so as to come into contact with the air leakage preventing member.

JP 2004 160444 A discloses an air conditioning rotating body having a rotor in a cylindrical shape, the rotor being housed in a casing so as to be freely rotatable, the air conditioning rotating body being configured to treat air passing through the rotor in an axial direction, wherein a labyrinth seal structure is provided between an outer circumferential portion of the rotor and the casing, and the labyrinth seal structure includes a first protrusion protruding in the axial direction from the rotor toward the casing, and a second protrusion protruding in the axial direction from the casing toward the rotor.

US 2004/231179 A1 addresses the problem of wear and particle generation due to a sliding contact between sealing members in an adsorption rotor and provides the solution of applying a labyrinth sealing structure.

### CITATION LIST

### PATENT DOCUMENT

Patent Document 1: Japanese Unexamined Patent Publication No. 2006-052922

### SUMMARY OF THE INVENTION

### TECHNICAL PROBLEM

An air treatment device using a rotor is configured to have a plurality of air passages where flows of air passing through the rotor in the axial direction are opposite to each other to achieve high performance. In this configuration, the pressure distribution in the casing changes depending on the rotational angle of the rotor. Thus, air leakage occurs from the center toward the outer circumference of the rotor and from the outer circumference toward the center of the rotor.

However, in the known sealing structure disclosed in Patent Document 1, the sealing performance deteriorates when the leakage air flows in a direction separating the air leakage preventing member and the receiving member from each other. In addition, if the shaft is displaced in installation of the rotor, great fluctuations in the axial direction occur at the outer circumferential portion of the rotor, resulting in a strong collision between the air leakage preventing member and the receiving member, which may cause damage of the members and loss of sealing performance.

An objective of the present disclosure is to reduce air leakage at an outer circumferential portion of a rotor which serves as an air conditioning rotating body.

### SOLUTION TO THE PROBLEM

The present disclosure is generally directed to an air conditioning body according to claim 1.

A first aspect of the present disclosure is directed to an air conditioning rotating body (30) having a rotor (10) in a cylindrical shape, the rotor (10) being housed in a casing (50) so as to be freely rotatable, the air conditioning rotating body (30) being configured to treat air passing through the rotor (10) in an axial direction, wherein a labyrinth seal structure (100) is provided between an outer circumferential portion of the rotor (10) and the casing (50), and the labyrinth seal structure (100) includes a first protrusion (23a) protruding in the axial direction from the rotor (10) toward the casing (50), and a second protrusion (56a) protruding in the axial direction from the casing (50) toward the rotor (10).

According to the first aspect, the labyrinth seal structure (100) provided at the outer circumferential portion of the rotor (10) can reduce both of the air leakage from the center toward the outer circumference of the rotor and the air leakage from the outer circumference toward the center of the rotor. Further, the labyrinth seal structure (100) is configured by the engagement between the protrusions (23a) and (56a) protruding in the rotor axial direction. This configuration makes it easier to maintain sealing performance even if the outer circumferential portion of the rotor (10) fluctuates in the axial direction due to displacement of the shaft, for example.

A second aspect of the present disclosure is directed to the air conditioning rotating body of the first aspect, in which at least one of the first protrusion (23a) or the second protrusion (56a) is an elastic element.

According to the second aspect, the wind pressure of leaking air causes the protrusion made of an elastic element to deform to become closer to the other protrusion. The sealing performance can thus be improved.

A third aspect of the present disclosure is directed to the air conditioning rotating body of the second aspect, in which the labyrinth seal structure (100) includes a plurality of engagement structures, and the plurality of engagement structures includes a plurality of the elastic elements inclined at different angles to the axial direction.

According to the third aspect, the labyrinth seal structure (100) can be optimized by adjusting the inclination angle of each elastic element according to the condition of leaking air.

A fourth aspect of the present disclosure is directed to the air conditioning rotating body of the third aspect, in which the plurality of engagement structures includes a plurality of the elastic elements further apart from each other toward ends of the elastic elements.

According to the fourth aspect, the end of the protrusion made of any one of the elastic elements becomes much closer to another protrusion even when the air leakage flows from the center toward the outer circumference of the rotor or from the outer circumference to the center of the rotor. The sealing performance can thus be further improved.

A fifth aspect of the present disclosure is directed to the air conditioning rotating body of any one of the second to fourth aspects, in which the first protrusion (23a) is an inelastic element, and the second protrusion (56a) is an elastic element.

The second protrusion (56a) protruding from the casing (50) is more likely to be affected by the wind pressure of leaking air than the first protrusion (23a) protruding from the rotor (10). Therefore, according to the fifth aspect, the second protrusion (56a) is made of an elastic element, which makes the second protrusions (56a) become much closer to the first protrusions (23a). The sealing performance can thus be further improved.

A sixth aspect of the present disclosure is directed to an air treatment device having the air conditioning rotating body (30) of any one of the first to fifth aspects.

The air treatment device of the sixth aspect has the air conditioning rotating body (30) that can reduce the air leakage at the outer circumferential portion of the rotor (10). This can improve the performance of the air treatment device (1), such as dehumidification and humidification performance and heat exchange performance.

### BRIEF DESCRIPTION OF THE DRAWINGS

FIG. 1 is a schematic view illustrating a general configuration of an air treatment device according to an embodiment.
FIG. 2 is a schematic view illustrating a cross-sectional configuration of an air conditioning rotating body according to the embodiment.
FIG. 3 is a perspective view of a rotor constituting the air conditioning rotating body according to the embodiment.
FIG. 4 is a perspective view of a protective case for accommodating the rotor illustrated in FIG. 3.
FIG. 5 is a perspective view illustrating how the rotor illustrated in FIG. 3 is accommodated in the protective case illustrated in FIG. 4.
FIG. 6 is a perspective view illustrating the state in which the rotor illustrated in FIG. 3 is accommodated in the protective case illustrated in FIG. 4.
FIG. 7 is a perspective view illustrating the state in which the air conditioning rotating body according to the embodiment is accommodated in a casing, with part of the figure cut out.
FIG. 8 is a schematic view illustrating a cross-sectional configuration of a labyrinth seal structure provided in the air conditioning rotating body according to the embodiment.
FIG. 9 is a schematic view illustrating an example of the cross-sectional configuration of the air conditioning rotating body according to the embodiment.
FIG. 10A is a schematic view illustrating an example of how the labyrinth seal structure provided in the air conditioning rotating body according to the embodiment reduces air leakage.
FIG. 10B is a schematic view illustrating an example of how the labyrinth seal structure provided in the air conditioning rotating body according to the embodiment reduces air leakage.
FIG. 10C is a schematic view illustrating an example of how the labyrinth seal structure provided in the air conditioning rotating body according to the embodiment reduces air leakage.
FIG. 11 is a schematic view illustrating a cross-sectional configuration of an air conditioning rotating body according to a first variation.
FIG. 12 is a schematic view illustrating a cross-sectional configuration of an air conditioning rotating body according to a second variation.

### DESCRIPTION OF EMBODIMENTS

### (Embodiment)

An embodiment of the present disclosure will be described in detail below with reference to the drawings. The present disclosure is not limited to the embodiment shown below, and various changes can be made within the scope of the appended claims. Since each of the drawings is intended to illustrate the disclosure conceptually, dimensions, ratios, or numbers may be exaggerated or simplified as necessary for ease of understanding.

### <Configuration of Air Treatment Device>

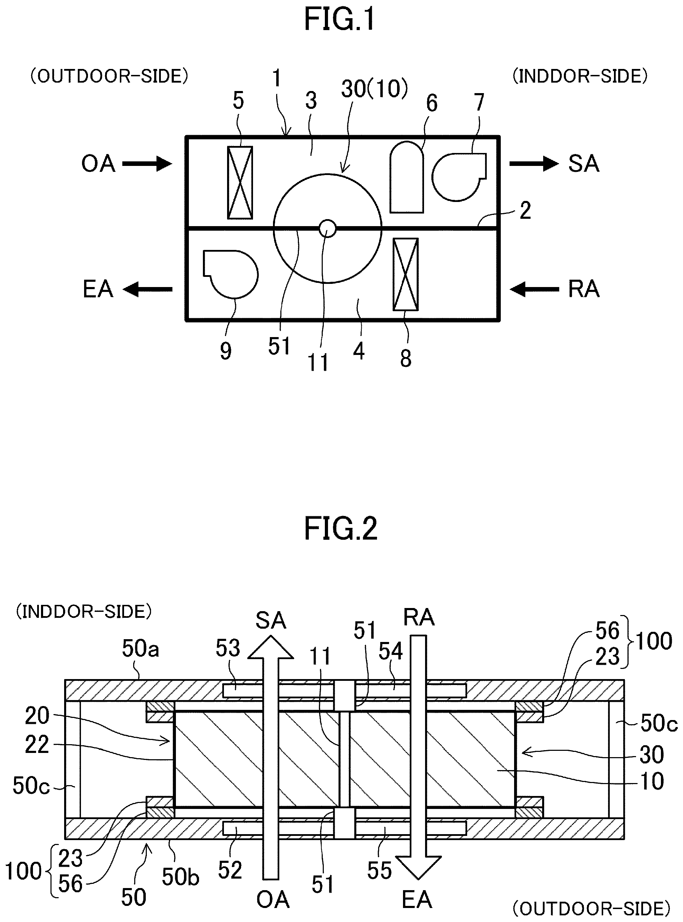

An air treatment device (1) of this embodiment is configured as a dehumidifying/humidifying device, for example. As illustrated in FIG. 1, the interior of the air treatment device (1) includes air passages (3) and (4) that are separated by a division wall (2). In the air treatment device (1), outdoor air (OA) taken from an outdoor space is supplied to an indoor space as supply air (SA) via the air passage (3), and return air (RA) taken from the indoor space is discharged to the outdoor space as exhaust air (EA) via the air passage (4).

An air conditioning rotating body (30) of this embodiment has a cylindrical rotor (10) made of a honeycomb-shaped adsorption element on which zeolite, for example, or another substance is supported, and a shaft (11) inserted in the center of the rotor (10). The shaft (11) is arranged on the boundary between the air passages (3) and (4). In other words, the air conditioning rotating body (30) is arranged over both of the air passages (3) and (4). The air passages (3) and (4) are each configured so that air flows in opposite directions and passes through the rotor (10) in its axial direction. Seal members (51), which separate the air passages (3) and (4) from each other, are slidably in contact with both axial end faces of the air conditioning rotating body (30).

In the air passage (3), a heat exchanger (5) is arranged upstream (outdoor-side) of the air conditioning rotating body (30), and a compressor (6) and a fan (7) are sequentially arranged downstream (indoor-side) of the air conditioning rotating body (30). In the air passage (4), a heat exchanger (8) is arranged upstream (indoor-side) of the air conditioning rotating body (30), and a fan (9) is arranged downstream (outdoor-side) of the air conditioning rotating body (30).

In a dehumidifying operation of the air treatment device (1), the rotor (10) is rotated to allow an adsorbent of the rotor (10) to adsorb water vapor from the air passing through the air passage (3), thereby generating dehumidified air. On the other hand, the adsorbent is regenerated by desorbing the water vapor from the adsorbent of the rotor (10) by air that is heated to a predetermined temperature and passes through the air passage (4). In this case, the heat exchanger (5) in the air passage (3) serves as an evaporator, and the heat exchanger (8) in the air passage (4) serves as a condenser.

In a humidifying operation of the air treatment device (1), the rotor (10) is rotated to allow the adsorbent of the rotor (10) to adsorb water vapor from the air passing through the air passage (4). On the other hand, humidified air is generated, and the adsorbent is regenerated, by desorbing the water vapor from the adsorbent of the rotor (10) by air that is heated to a predetermined temperature and passes through the air passage (3). In this case, the heat exchanger (5) in the air passage (3) serves as a condenser, and the heat exchanger (8) in the air passage (4) serves as an evaporator.

### <Configuration of Air Conditioning Rotating Body>

In the air treatment device (1), the air conditioning rotating body (30) is housed in a casing (50) so as to be freely rotatable as illustrated in FIG. 2. The air conditioning rotating body (30) includes a protective case (20) that protects the rotor (10). The protective case (20) includes a side plate (22) that covers the side surface on the outer circumference of the rotor (10). The protective case (20) rotates about the shaft (11) together with the rotor (10).

The casing (50) includes a substantially square-shaped upper plate (50a) and lower plate (50b), which face each other in the axial direction of the rotor (10) with the air conditioning rotating body (30) interposed therebetween, and supports (50c) that connect the upper plate (50a) and the lower plate (50b) at the respective four corners. The upper plate (50a) has an exhaust port (53) connected to the downstream side of the air passage (3) and an intake port (54) connected to the upstream side of the air passage (4). The lower plate (50b) has an intake port (52) connected to the upstream side of the air passage (3) and an exhaust port (55) connected to the downstream side of the air passage (4). The intake ports (52) and (54) and the exhaust ports (53) and (55) each have a substantially semicircular shape when viewed from the axial direction of the rotor (10). The intake port (52) of the lower plate (50b) and the exhaust port (53) of the upper plate (50a) overlap each other when viewed from the axial direction of the rotor (10). The intake port (54) of the upper plate (50a) and the exhaust port (55) of the lower plate (50b) overlap each other when viewed from the axial direction of the rotor (10).

The seal members (51) are arranged at the boundary between the exhaust port (53) and the intake port (54) in the upper plate (50a) and at the boundary between the intake port (52) and the exhaust port (55) in the lower plate (50b). The seal members (51) are slidably in contact with respective axial end faces of the air conditioning rotating body (30). This configuration can reduce leakage of air, which is sucked from the intake port (52) of the lower plate (50b), leaking to the exhaust port (55) of the lower plate (50b) through the space between the rotor (10) and the lower plate (50b) without passing through the rotor (10). This configuration can also reduce leakage of air, which is sucked from the intake port (54) of the upper plate (50a), leaking to the exhaust port (53) of the upper plate (50a) through the space between the rotor (10) and the upper plate (50a) without passing through the rotor (10).

A rotor-side seal (23) is provided on each of upper and lower portions of the side surface on the outer circumference of the rotor (10), that is, the side plate (22) covering the outer circumferential portion of the rotor (10). A casing-side seal (56) is provided at a portion opposite to the rotor-side seal (23) on each of the upper plate (50a) and the lower plate (50b). The rotor-side seal (23) and the casing-side seal (56) engage with each other to constitute a labyrinth seal structure (100). In other words, the labyrinth seal structure (100) is provided between the outer circumferential portion of the rotor (10) and the casing (50). This configuration can reduce leakage of air, which is sucked from the intake port (52) of the lower plate (50b), leaking to the exhaust port (53) of the upper plate (50a) while bypassing the rotor (10). This configuration can also reduce leakage of air, which is sucked from the intake port (54) of the upper plate (50a), leaking to the exhaust port (55) of the lower plate (50b) while bypassing the rotor (10). Details of the labyrinth seal structure (100) will be described later.

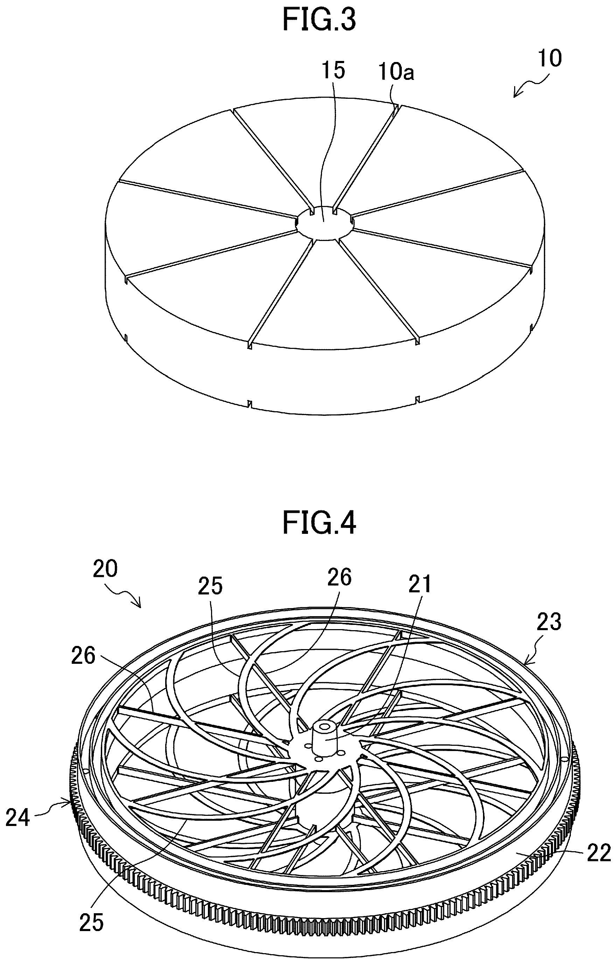

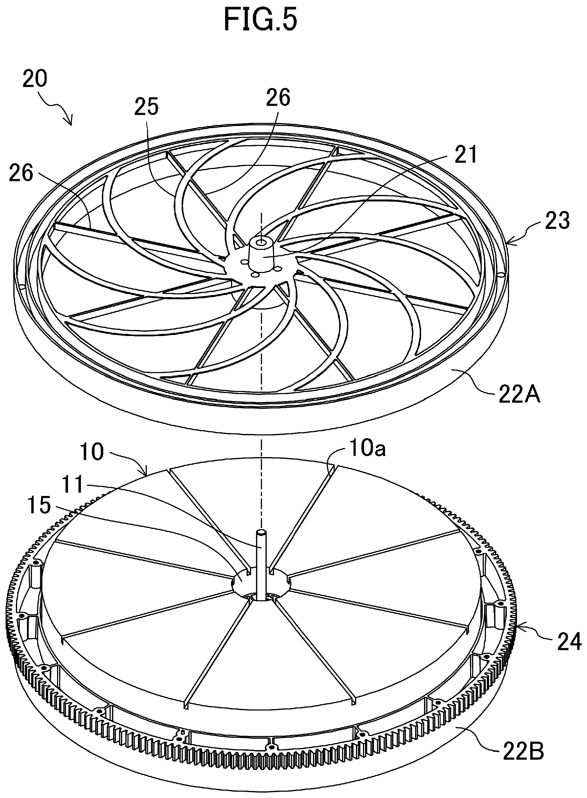

In this embodiment, the rotor (10) is a honeycomb-shaped adsorption element on which zeolite, for example, or another substance is supported, and is configured as a cylindrical body with a through hole (15) in the center, as illustrated in FIG. 3. The protective case (20) that protects the rotor (10) has an inner cylinder (21) fitted in the through hole (15) of the rotor (10) and supporting the shaft (11), and the annular side plate (22) that covers the side surface on the outer circumference of the rotor (10), as illustrated in FIGS. 4 to 6. The protective case (20) may be, for example, a resin molded product or may be made of sheet metal. The side plate (22) is separable into an upper side plate (22A) and a lower side plate (22B) for easy attachment and detachment of the protective case (20) to and from the rotor (10). The annular rotor-side seal (23) is provided on each of the upper and lower portions of the side plate (22). An annular gear (24) for rotating the air conditioning rotating body (30) by means of a motor and a small gear (both located outside the illustrated figures) is provided at an intermediate portion of a side surface on the outer circumference of the side plate (22). The inner cylinder (21) and the side plate (22) are connected to each other with spokes (25) and reinforcing ribs (26) which are in contact with respective axial end faces of the rotor (10). On each of the axial end faces of the rotor (10), open regions where the spokes (25) and the reinforcing ribs (26) are not provided serve as ventilation holes of the air conditioning rotating body (30). The spokes (25) may be curved in the circumferential direction of the rotor (10). The reinforcing ribs (26) may extend in the radial direction and may be fitted into the axial end faces of the rotor (10). The rotor (10) illustrated in FIG. 3 has grooves (10a) on the axial end faces. The grooves (10a) are for having part of the respective reinforcing ribs (26) fitted therein and extend along the radial direction.

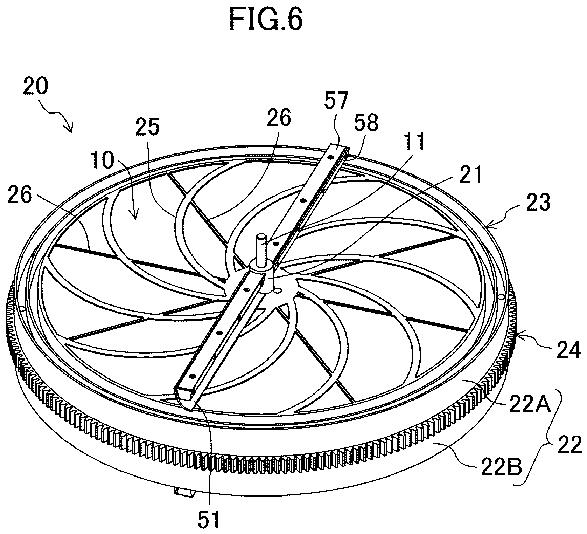

As illustrated in FIG. 6, the sheet-like seal members (51), which separates the air passages (3) and (4) from each other, are slidably in contact with the axial end faces of the air conditioning rotating body (30). Each of the seal members (51) is an elastic element, such as rubber, and is arranged along the radial direction of the rotor (10). The upper plate (50a) and the lower plate (50b) are each provided with supports (57) made of rubber, for example, for separating the intake port (52), (54) and the exhaust port (53), (55) from each other. The seal member (51) is held by being partially sandwiched between a side surface of the support (57) and a plate body (58). The supports (57) extend from the inner cylinder (21) of the protective case (20) in two directions opposite to each other by 180° along the radial direction.

In this embodiment, the air conditioning rotating body (30), i.e., rotor (10), is rotated with the spokes (25) arranged on each axial end face of the rotor (10); therefore, direct contact of the rotor (10) with the seal members (51) can be avoided due to the interposition of the spokes (25). Wear of the rotor (10) can thus be reduced. Wear of the seal members (51) can also be reduced by using a material having higher sliding properties than the material of the surface of the rotor (10) as the material of the spokes (25), that is, the protective case (20).

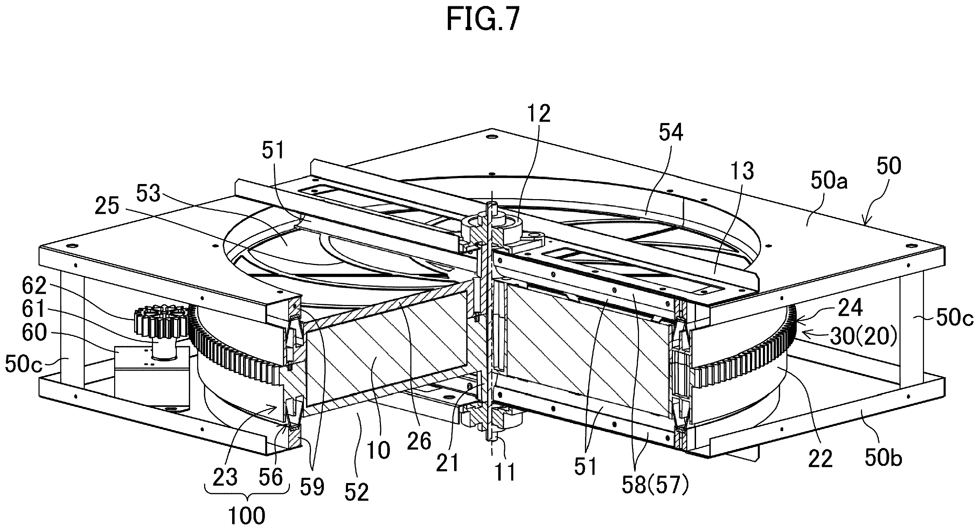

The air conditioning rotating body (30) is housed in the casing (50) so as to be freely rotatable as illustrated in FIG. 7. A motor (60) and a small gear (62) connected to a shaft (61) of the motor (60) are provided on the lower plate (50b) of the casing (50) near the air conditioning rotating body (30). The small gear (62), when rotated by the motor (60), drives and rotates the annular gear (24) on the side plate (22) covering the side surface on the outer circumference of the rotor (10), causing the air conditioning rotating body (30) to rotate. The shaft (11) of the air conditioning rotating body (30), i.e., the rotor (10), is fixed to, and supported by, the inner cylinder (21) of the protective case (20), and portions of the shaft (11) protruding from upper and lower ends of the inner cylinder (21) are rotatably held by bearing portions (12). On a surface of each of the upper plate (50a) and the lower plate (50b) opposite to the surface facing the air conditioning rotating body (30), a bearing support (13) is provided along the boundary between the intake port (52), (54) and the exhaust port (53), (55). The bearing portions (12) are attached to the respective bearing supports (13). The supports (57) holding the seal members (51) are attached to a surface of the bearing support (13) facing the air conditioning rotating body (30).

An annular support (59) made of resin, for example, is provided at each of the peripheral portion of the exhaust port (53) and the intake port (54) of the upper plate (50a) and the peripheral portion of the intake port (52) and the exhaust port (55) of the lower plate (50b). The casing-side seal (56) is attached to a surface of the annular support (59) facing the air conditioning rotating body (30). The end face of the support (57) on the outer circumference side of the rotor (10) is connected to an inner circumferential surface of annular support (59).

### <Labyrinth Seal Structure>

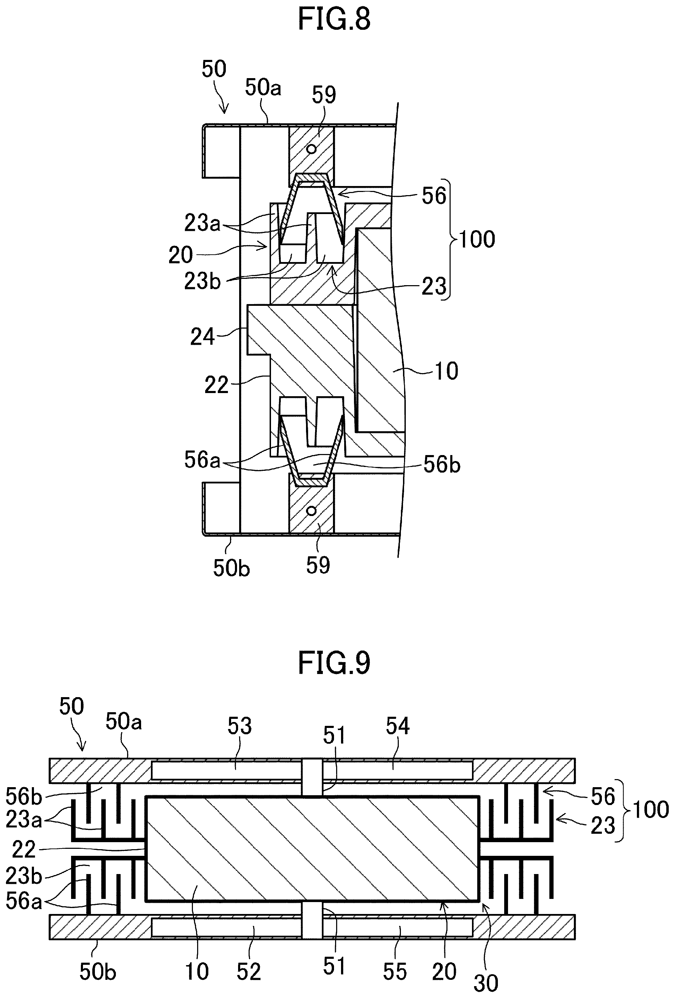

As illustrated in FIGS. 8 and 9, the rotor-side seal (23) is provided on each of the upper and lower portions of the side plate (22) covering the side surface on the outer circumference of the rotor (10). The casing-side seal (56) is provided on the annular support (59) provided on each of the upper plate (50a) and the lower plate (50b). The rotor-side seal (23) and the casing-side seal (56) engage with each other to constitute the labyrinth seal structure (100). **In** this configuration, the labyrinth seal structure (100) is provided between the outer circumferential portion of the rotor (10) and the casing (50). **In** FIG. 9, the annular supports (59) are shown integrally with the upper plate (50a) and lower plate (50b).

The rotor-side seal (23) has a plurality of first protrusions (23a) protruding in the axial direction of the rotor (10) toward the casing (50) and a first recess (23b) between adjacent first protrusions (23a). The casing-side seal (56) has a plurality of second protrusions (56a) protruding in the axial direction of the rotor (10) toward the rotor (10) and a second recess (56b) between adjacent second protrusions (56a).

As an example, in this embodiment, the rotor-side seal (23) has three first protrusions (23a) and two first recesses (23b), and the casing-side seal (56) has two second protrusions (56a) and one second recess (56b). The first protrusions (23a) and the second protrusions (56a) are each arranged concentrically around the center (shaft (11)) of the rotor (10). The middle one of the three first protrusions (23a) is inserted in the second recess (56b). Each of the second protrusions (56a) is inserted in the corresponding one of the first recesses (23b). The annular labyrinth seal structure (100) including a two-stage engagement structure is formed in this manner. The number of stages of the engagement structure in the labyrinth seal structure (100) is not limited, and may be one stage or may be three or more stages.

The gap (distance in the radial direction of the rotor (10)) between the first protrusion (23a) and the second protrusion (56a) in the labyrinth seal structure (100) may be set to about 1 mm, for example. This gap forms an annular zigzag channel (non-contact labyrinth structure) between the rotor-side seal (23) and the casing-side seal (56), increasing the resistance of air passing through the channel. The air leakage amount can thus be reduced. The labyrinth seal structure (100) can reduce both of the air leakage from the center toward the outer circumference of the rotor and the air leakage from the outer circumference toward the center of the rotor.

Although not particularly limited, the length of each of the first protrusion (23a) and the second protrusion (56a) may be, for example, about 30 mm to 40 mm, and the overlap length between them may be, for example, about 20 mm to 30 mm, in the labyrinth seal structure (100). This configuration can reduce damage of the rotor-side seal (23) and the casing-side seal (56) due to collision between them, even when the shaft of the rotor (10) is displaced and fluctuations in the axial direction occur at the outer circumferential portion of the rotor (10). Specifically, the sealing structure can be maintained while reducing collision of the first protrusions (23a) of the rotor-side seal (23) with the casing-side seal (56) and collision of the second protrusions (56a) of the casing-side seal (56) with the rotor-side seal (23).

At least either the first protrusions (23a) or the second protrusions (56a) may be elastic elements. For example, the first protrusions (23a) may be inelastic elements made of such as resin, and the second protrusions (56a) may be elastic elements made of such as rubber. In this case, the labyrinth seal structure (100) is configured by alternately arranging, in the radial direction of the rotor (10), the second protrusions (56a), which are elastic elements, and the first protrusions (23a), which are inelastic elements. As illustrated in FIG. 8, base portions of the elastic elements to be the two second protrusions (56a) may be connected to each other, and the connected portion may be fixed to the annular support (59).

The inelastic elements or the elastic elements to be the first protrusions (23a) and the second protrusions (56a) may have surface asperities with geometries of lateral lines, vertical lines, diagonal lines, or projections, for example. Alternatively, the inelastic elements or the elastic elements to be the first protrusions (23a) and the second protrusions (56a) may have surface asperities made with a brush or fibers. This configuration can achieve a more complicated labyrinth seal structure (100), thereby improving the sealing performance.

If the labyrinth seal structure (100) includes a plurality of engagement structures, as in this embodiment, the plurality of engagement structures may include a plurality of elastic elements (second protrusions (56a)) inclined at different angles to the axial direction of the rotor (10), as illustrated in FIG. 8. In FIG. 8, the second protrusion (56a) closer to the rotor (10) is inclined so that its end is closer to the rotor (10), and the second protrusion (56a) farther from the rotor (10) is inclined so that its end is away from the rotor (10). In other words, the two second protrusions (56a), which are elastic elements, are further apart toward their respective ends. The clearance (distance in the radial direction of the rotor (10)) between the end of each second protrusion (56a) and the end of the adjacent first protrusion (23a) may be set to about 1 mm, for example.

Instead of the configuration of the casing-side seal (56) illustrated in FIG. 8, the second protrusion (56a) closer to the rotor (10) may be inclined so that its end is away from the rotor (10), and the second protrusion (56a) farther from the rotor (10) may be inclined so that its end is closer to the rotor (10). In other words, the two second protrusions (56a), which are elastic elements, are closer to each other toward their respective ends.

### <Advantages of Embodiment>

According to the air conditioning rotating body (30) of this embodiment, the labyrinth seal structure (100) is provided between the outer circumferential portion of the rotor (10) and the casing (50). Thus, the labyrinth seal structure (100) can reduce both of the air leakage from the center toward the outer circumference of the rotor and the air leakage from the outer circumference toward the center of the rotor.

According to the air conditioning rotating body (30) of this embodiment, the labyrinth seal structure (100) includes the first protrusion (23a) protruding in the axial direction of the rotor (10) toward the casing (50), and the second protrusion (56a) protruding in the axial direction of the rotor (10) toward the rotor (10). In other words, the labyrinth seal structure (100) is configured by the engagement between the protrusions (23a) and (56a) protruding in the rotor axial direction. This configuration makes it easier to maintain sealing performance even if the outer circumferential portion of the rotor (10) fluctuates in the axial direction due to displacement of the shaft, for example.

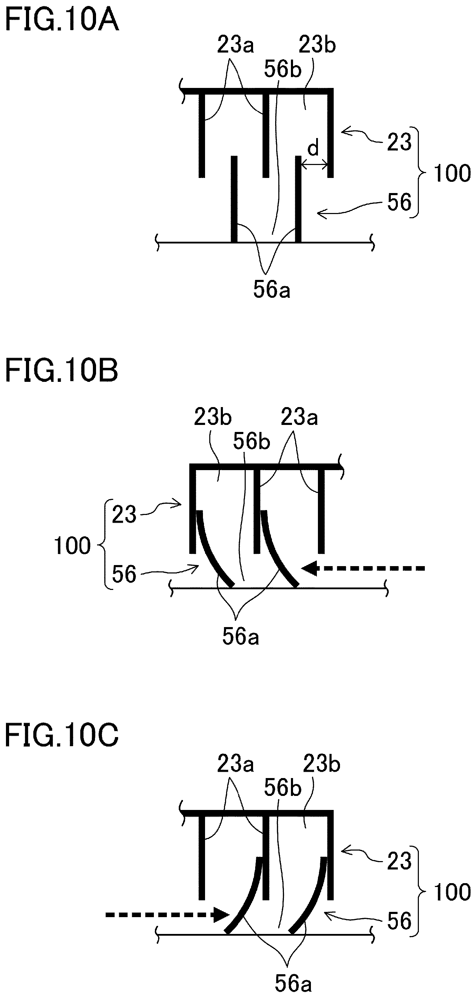

FIG. 10A is a diagram illustrating a state in which the outer circumferential portion of the rotor (10) fluctuates in the axial direction due to displacement of the shaft, causing the rotor-side seal (23) and the casing-side seal (56) constituting the labyrinth seal structure (100) to be further apart, compared to a case where there is no displacement of the shaft. Even in the state illustrated in FIG. 10A, the engagement structure between the rotor-side seal (23) and the casing-side seal (56) is maintained, and the distance d between the first protrusion (23a) and the second protrusion (56a) (distance between seals) is secured, so that the sealing performance can be maintained without wear of the seals (23) and (56).

At least either the first protrusions (23a) or the second protrusions (56a) may be elastic elements in the air conditioning rotating body (30) of this embodiment. In this configuration, the wind pressure of leaking air causes the protrusions made of elastic elements to deform to become closer to the other protrusions. The sealing performance can thus be improved.

If at least either the first protrusions (23a) or the second protrusions (56a) are elastic elements, the labyrinth seal structure (100) may include a plurality of engagement structures, and the plurality of engagement structures may include a plurality of elastic elements inclined at different angles to the axial direction of the rotor (10). In this configuration, the labyrinth seal structure (100) can be optimized by adjusting the inclination angle of each elastic element according to the condition of leaking air. In this case, the plurality of elastic elements may be further apart from each other toward the respective ends. In this configuration, the ends of either protrusions that are made of elastic elements become much closer to the other protrusions even when the air leakage flows from the center toward the outer circumference of the rotor or from the outer circumference to the center of the rotor. The sealing performance can thus be further improved. Similar effects are obtainable also when the plurality of elastic elements are arranged to be closer to each other toward the respective ends. The plurality of elastic elements may include an elastic element inclined in one of the radial directions and an elastic element inclined in the other radial direction, with respect to the axial direction of the rotor (10).

If at least either the first protrusions (23a) or the second protrusions (56a) are elastic elements, the first protrusions (23a) may be inelastic elements, and the second protrusions (56a) may be elastic elements. In the case where the rotor-side seal (23) is provided on the side plate (22) covering the side surface on the outer circumference of the rotor (10) as illustrated in FIG. 9, the pressure of air leaking through the gap between the casing (50) and the rotor (10) (air conditioning rotating body (30)) to the outer circumferential portion of the rotor (10) is greater on the second protrusions (56a) than on the first protrusions (23a) disposed behind the side plate (22). Therefore, if the second protrusions (56a) are elastic elements, the second protrusions (56a) become much closer to the first protrusions (23a). The sealing performance can thus be further improved.

FIGS. 10B and 10C are diagrams illustrating how the second protrusions (56a), which are elastic elements, are deformed to approach the first protrusions (23a), which are inelastic elements, by the wind pressure of the leaking air (indicated by the dashed arrows in the diagrams). As illustrated in FIGS. 10B and 10C, the deformation of the second protrusions (56a), which are elastic elements contributes to further reduction of both of the air leakage from the center toward the outer circumference of the rotor and the air leakage from the outer circumference toward the center of the rotor.

The air treatment device (1) of this embodiment has the air conditioning rotating body (30) that can reduce the air leakage at the outer circumferential portion of the rotor (10). Thus, the performance of the air treatment device (1), such as dehumidification and humidification performance and heat exchange performance, can be improved.

### (First Variation)

A first variation of the present disclosure will be described below with reference to the drawings.

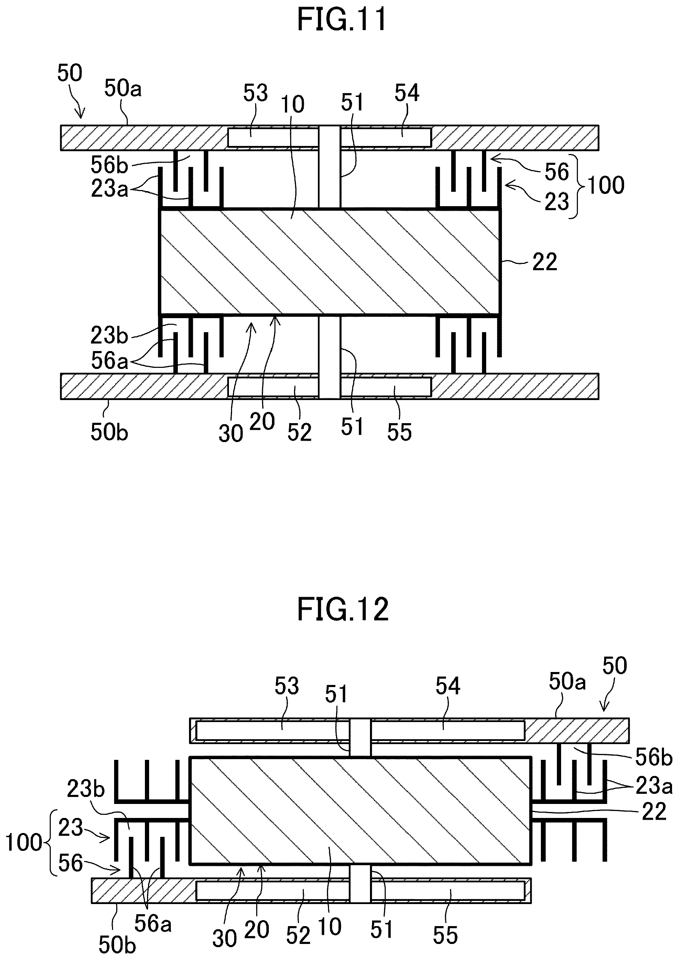

In the above embodiment, the rotor-side seal (23) is provided on the side plate (22) covering the side surface on the outer circumference of the rotor (10), as illustrated in FIG. 9. In this first variation, however, the rotor-side seal (23) is provided on an outer circumferential portion of each axial end face of the rotor (10), as illustrated in FIG. 11. In FIG. 11, the same reference characters are used to designate the same elements as those in the embodiment illustrated in FIG. 9.

As illustrated in FIGS. 9 and 11, the axial dimension of the rotor (10) in the air conditioning rotating body (30) can be reduced in the configuration of the aforementioned embodiment, whereas the radial dimension of the rotor (10) in the air conditioning rotating body (30) can be reduced in the configuration of this first variation.

### (Second Variation)

A second variation of the present disclosure will be described below with reference to the drawings.

In the above embodiment, the casing-side seal (56) is provided on the entire outer circumferential portion of the rotor (10), as illustrated in FIG. 9. In the second variation, however, the casing-side seal (56) is provided on part of the outer circumferential portion, for example, on the halfway around the outer circumferential portion, of the rotor (10), as illustrated in FIG. 12. Specifically, in order to reduce leakage of air, which is sucked from the intake port (52) of the lower plate (50b), leaking to the exhaust port (53) of the upper plate (50a) while bypassing the rotor (10), the casing-side seal (56) is provided around the intake port (52) of the lower plate (50b), but is not provided around the exhaust port (55) of the lower plate (50b). Further, in order to reduce leakage of air, which is sucked from the intake port (54) of the upper plate (50a), leaking to the exhaust port (55) of the lower plate (50b) while bypassing the rotor (10), the casing-side seal (56) is provided around the intake port (54) of the upper plate (50a), but is not provided around the exhaust port (53) of the upper plate (50a). In FIG. 12, the same reference characters are used to designate the same elements as those in the embodiment illustrated in FIG. 9.

Compared to the configuration of the above embodiment, the configuration of this second variation can reduce the quantity of seal members for use in the labyrinth seal structure (100) (casing-side seal (56)), thereby reducing manufacturing costs.

### (Other Embodiments)

In the foregoing embodiment (including the variations, hereinafter the same), the air treatment device (1) is configured as a dehumidifying/humidifying device, by using, as the rotor (10) of the air conditioning rotating body (30), a honeycomb-shaped adsorption element on which zeolite is supported. However, instead of this configuration, for example, the air treatment device (1) may be configured as a deodorizer, a gas separator, or the like, by using an air conditioning rotating body with a rotor that is a honeycomb-shaped adsorption element on which another adsorbent, such as porous silica or activated alumina, is supported. Alternatively, for example, the air treatment device (1) may be configured as a heat exchanger, by using an air conditioning rotating body with a rotor made of a material with excellent heat storage properties, such as aluminum or stainless steel.

In the above embodiment, the air conditioning rotating body (30) is arranged in the air treatment device (1) such that the radial direction of the rotor (10) is along the horizontal direction. However, instead of this configuration, the air conditioning rotating body (30) may be arranged in the air treatment device (1) such that the radial direction of the rotor (10) is along the perpendicular (vertical) direction.

In the above embodiment, the two air passages (3) and (4) are formed in the air treatment device (1), and the air conditioning rotating body (30) is arranged over both of the air passages (3) and (4). However, the number of air passages in the air treatment device (1) (i.e., the number of air passages where the air conditioning rotating body (30) is arranged) is not particularly limited, and may be one or may be three or more. If one air passage is formed in the air treatment device (1), and the air flow direction in this air passage changes with time, the air conditioning rotating body (30) of this disclosure can reduce both of the air leakage from the center toward the outer circumference of the rotor and the air leakage from the outer circumference toward the center of the rotor.

While the embodiments and variations have been described above, it will be understood that various changes in form and details can be made without departing from the scope of the claims.

### INDUSTRIAL APPLICABILITY

As can be seen from the foregoing description, the present disclosure is useful for an air conditioning rotating body and an air treatment device.

### DESCRIPTION OF REFERENCE CHARACTERS

- 1: Air Treatment Device
- 10: Rotor
- 23a: First Protrusion
- 30: Air Conditioning Rotating Body
- 50: Casing
- 56a: Second Protrusion
- 100: Labyrinth Seal Structure

## Claims

1. An air conditioning rotating body (30) having a rotor (10) in a cylindrical shape, the rotor (10) being housed in a casing (50) so as to be freely rotatable, the air conditioning rotating body (30) being configured to treat air passing through the rotor (10) in an axial direction, wherein
a labyrinth seal structure (100) is provided between an outer circumferential portion of the rotor (10) and the casing (50),
the labyrinth seal structure (100) includes a first protrusion (23a) protruding in the axial direction from the rotor (10) toward the casing (50), and a second protrusion (56a) protruding in the axial direction from the casing (50) toward the rotor (10),
one of the first protrusion (23a) or the second protrusion (56a) is an elastic element configured to be deformed by a wind pressure of air leaking to a space between the outer circumferential portion of the rotor (10) and the casing (50),
the other of the first protrusion (23a) or the second protrusion (56a) is an inelastic element that is not deformed by the wind pressure of air leaking to the space between the outer circumferential portion of the rotor (10) and the casing (50),
the first protrusion (23a) includes a plurality of first protrusions (23a), and the second protrusion (56a) includes a plurality of second protrusions (56a),
the labyrinth seal structure (100) includes: the plurality of first protrusions (23a); a first recess (23b) between adjacent ones of the plurality of first protrusions (23a); the plurality of second protrusions (56a); and a second recess (56b) between adjacent ones of the plurality of second protrusions (56a), and
a corresponding one of the plurality of first protrusions (23a) is inserted in the second recess (56b) and a corresponding one of the plurality of second protrusions (56a) is inserted in the first recess (23b).

2. The air conditioning rotating body of claim 1, wherein
the first protrusion (23a) is the inelastic element, and
the second protrusion (56a) is the elastic element.

3. The air conditioning rotating body of claim 2, wherein
the labyrinth seal structure (100) includes the plurality of second protrusions (56a) inclined at angles different from each other to the axial direction.

4. The air conditioning rotating body of claim 3, wherein
the labyrinth seal structure (100) includes the plurality of second protrusions (56a) further apart from each other toward ends of the second protrusions (56a).

5. An air treatment device (1) comprising the air conditioning rotating body (30) of any one of claims 1 to 4, wherein
air passages (3) and (4) separated from each other by a division wall (2) are provided in the air treatment device (1), and
the air conditioning rotating body (30) is arranged over both of the air passages (3) and (4).

## Patentansprüche

1. Klimaanlagendrehkörper (30) mit einem Rotor (10) in einer zylindrischen Form, wobei der Rotor (10) in einem Gehäuse (50) untergebracht ist, um frei drehbar zu sein, wobei der Klimaanlagendrehkörper (30) konfiguriert ist, um Luft zu behandeln, die durch den Rotor (10) in einer axialen Richtung strömt, wobei
eine Labyrinthdichtungsstruktur (100) zwischen einem Außenumfangsabschnitt des Rotors (10) und dem Gehäuse (50) vorgesehen ist,
die Labyrinthdichtungsstruktur (100) einen ersten Vorsprung (23a), der in der axialen Richtung von dem Rotor (10) in Richtung des Gehäuses (50) vorsteht, und einen zweiten Vorsprung (56a), der in der axialen Richtung von dem Gehäuse (50) in Richtung des Rotors (10) vorsteht, enthält,
einer von dem ersten Vorsprung (23a) oder dem zweiten Vorsprung (56a) ein elastisches Element ist, das konfiguriert ist, um durch einen Winddruck von Luft, die in einen Raum zwischen dem Außenumfangsabschnitt des Rotors (10) und dem Gehäuse (50) austritt, verformt zu werden,
der andere von dem ersten Vorsprung (23a) oder dem zweiten Vorsprung (56a) ein unelastisches Element ist, das nicht durch den Winddruck von Luft, die in den Raum zwischen dem Außenumfangsabschnitt des Rotors (10) und dem Gehäuse (50) austritt, verformt wird,
der erste Vorsprung (23a) eine Vielzahl von ersten Vorsprüngen (23a) umfasst und der zweite Vorsprung (56a) eine Vielzahl von zweiten Vorsprüngen (56a) enthält,
die Labyrinthdichtungsstruktur (100) enthält: die Vielzahl von ersten Vorsprüngen (23a); eine erste Aussparung (23b) zwischen benachbarten von der Vielzahl von ersten Vorsprüngen (23a); die Vielzahl von zweiten Vorsprüngen (56a); und eine zweite Aussparung (56b) zwischen benachbarten von der Vielzahl von zweiten Vorsprüngen (56a), und
ein entsprechender von der Vielzahl von ersten Vorsprüngen (23a) in die zweite Aussparung (56b) eingesetzt ist und ein entsprechender von der Vielzahl von zweiten Vorsprüngen (56a) in die erste Aussparung (23b) eingesetzt ist.

2. Klimaanlagendrehkörper nach Anspruch 1, wobei
der erste Vorsprung (23a) das unelastische Element ist, und
der zweite Vorsprung (56a) das elastische Element ist.

3. Klimaanlagendrehkörper nach Anspruch 2, wobei
die Labyrinthdichtungsstruktur (100) die Vielzahl von zweiten Vorsprüngen (56a) enthält, die in voneinander verschiedenen Winkeln zu der axialen Richtung geneigt sind.

4. Klimaanlagendrehkörper nach Anspruch 3, wobei
die Labyrinthdichtungsstruktur (100) die Vielzahl von zweiten Vorsprüngen (56a) weiter voneinander entfernt in Richtung von Enden der zweiten Vorsprünge (56a) enthält.

5. Luftbehandlungsvorrichtung (1), die den Klimaanlagendrehkörper (30) nach einem der Ansprüche 1 bis 4 umfasst, wobei
Luftdurchgänge (3) und (4), die durch eine Trennwand (2) voneinander getrennt sind, in der Luftbehandlungsvorrichtung (1) vorgesehen sind, und
der Klimaanlagendrehkörper (30) über beiden der Luftdurchgänge (3) und (4) angeordnet ist.

## Revendications

1. Corps rotatif de climatisation (30) possédant un rotor (10) de forme cylindrique, le rotor (10) étant contenu dans un boîtier (50) de manière à pouvoir tourner librement, le corps rotatif de climatisation (30) étant configuré pour assurer le traitement de l'air traversant le rotor (10) dans une direction axiale,
une structure de joint labyrinthe (100) étant agencée entre une partie circonférentielle extérieure du rotor (10) et le boîtier (50),
la structure de joint labyrinthe (100) comprenant une première saillie (23a) dépassant dans la direction axiale du rotor (10) vers le carter (50), et une deuxième saillie (56a) dépassant dans la direction axiale du carter (50) vers le rotor (10),
l'une de la première saillie (23a) ou de la deuxième saillie (56a) étant un élément élastique configuré pour être déformé par une pression de vent de l'air fuyant vers un espace entre la partie circonférentielle extérieure du rotor (10) et le boîtier (50),
l'autre de la première saillie (23a) ou de la deuxième saillie (56a) étant un élément inélastique que ne déforme pas la pression de vent de l'air fuyant vers l'espace entre la partie circonférentielle extérieure du rotor (10) et le boîtier (50),
la première saillie (23a) comprenant une pluralité de premières saillies (23a), et la deuxième saillie (56a) comprenant une pluralité de deuxièmes saillies (56a),
la structure de joint labyrinthe (100) comprenant : la pluralité de premières saillies (23a) ; un premier évidement (23b) entre des saillies adjacentes de la pluralité de premières saillies (23a) ; la pluralité de deuxièmes saillies (56a) ; et un deuxième évidement (56b) entre des saillies adjacentes de la pluralité de deuxièmes saillies (56a), et
une saillie correspondante de la pluralité de premières saillies (23a) étant insérée dans le deuxième évidement (56b), et une saillie correspondante de la pluralité de deuxièmes saillies (56a) étant insérée dans le premier évidement (23b).

2. Corps rotatif de climatisation selon la revendication 1,
la première saillie (23a) étant l'élément inélastique, et
la deuxième saillie (56a) étant l'élément élastique.

3. Corps rotatif de climatisation selon la revendication 2,
la structure de joint labyrinthe (100) comprenant la pluralité de deuxièmes saillies (56a) inclinées à des angles différents les uns des autres relativement à la direction axiale.

4. Corps rotatif de climatisation selon la revendication 3,
la structure de joint labyrinthe (100) comprenant la pluralité de deuxièmes saillies (56a) plus espacées les unes des autres vers les bouts des deuxièmes saillies (56a).

5. Dispositif de traitement d'air (1) comprenant le corps rotatif de climatisation (30) selon une quelconque des revendications 1 à 4,
des conduits d'air (3) et (4), séparés l'un de l'autre par une cloison (2), étant agencés dans le dispositif de traitement d'air (1), et
le corps rotatif de climatisation (30) étant agencé au-dessus des deux conduits d'air (3) et (4).
